# EUROPEAN PATENT APPLICATION

(11) **EP 3 821 797 A1**
(43) Date of publication of application: **19.05.2021**
(21) Application number: 19889107.9
(22) Date of filing: 28.11.2019
(51) Int. Cl.: A61B 5/022

(54) **ARTERIAL STIFFNESS DISPLAY METHOD, SYSTEM, AND APPARATUS**

(30) Priority: 29.11.2018 CN 201811446012
(71) Applicant: Dongguan Kangzhu Medical Technology Co., Ltd., Dongguan, Guangdong 523000 (CN)
(72) Inventor: WANG, Peng, Dongguan, Guangdong 523000 (CN); CHEN, Long, Dongguan, Guangdong 523000 (CN)
(74) Representative: Sach, Greg Robert
(86) International application number: PCT/CN2019/121532
(87) International publication number: WO 2020/108556

(57) **Abstract**

The present disclosure is applicable to the medical technical field, and provides an arterial stiffness display method, system (1) and apparatus (2). The arterial stiffness display apparatus including an arm belt (204), a pressure sensor (205) and a human-machine interaction device (209) acquires a pressure value applied by an artery to the pressure sensor (205) in a pressure rise or drop process of the arm belt (204) (S101), acquires a pressure characteristic curve of the pressure value changing over time (S102), and acquires, according to the characteristic of a curve segment, corresponding to the preset pressure value, in the pressure characteristic curve, the arterial stiffness index (S103), so that the operation is simple, a relatively accurate arterial stiffness index can be obtained, and the accuracy of arterial stiffness detection can be effectively improved. Blood pressure values and a pulse number are acquired according to the pressure value, and at most two of the blood pressure values and the pulse number, and the arterial stiffness index are displayed by means of the human-machine interaction device (209), so that various parameters can be simultaneously measured and displayed, and the apparatus is suitable for widespread popularization and use.

## Description

### TECHNICAL FIELD

The present disclosure belongs to the medical technical field, and particularly relates to an arterial stiffness display method, system and apparatus.

### BACKGROUD

With the continuous development of medical technologies and people's increasing attention to their own health status, the use of various medical equipment to detect corresponding physiological parameters has become a common way to detect the health status.

However, existing apparatuses used for detecting arterial stiffness are complicated in operation, and obtain relatively rough detection results, so that they are difficult to popularize and use.

### SUMMARY

### Technical problem

One of the objectives of the embodiments of the present disclosure is to: provide an arterial stiffness display method, system and apparatus to solve the problems that since existing apparatuses used for detecting arterial stiffness are complicated in operation, and obtain relatively rough detection results, these apparatuses are difficult to popularize and use.

### Solutions of the problems

### Technical solutions

In order to solve the above technical problems, a first aspect of the embodiments of the present disclosure provides an arterial stiffness display method that is realized based on an arterial stiffness display apparatus. The arterial stiffness display apparatus includes an arm belt, a pressure sensor and a human-machine interaction device.

The arterial stiffness display method includes:
acquiring a pressure value of the pressure sensor in a pressure rise or drop process of the arm belt;
generating a pressure characteristic curve of the pressure value changing over time;
acquiring an arterial stiffness index according to a characteristic of a curve segment, corresponding to a preset pressure value, in the pressure characteristic curve;
acquiring blood pressure values and a pulse number according to the pressure value, the blood pressure values including a highest blood pressure value and a lowest blood pressure value; and
displaying at most two of the blood pressure values and the pulse number, and the arterial stiffness index by means of the human-machine interaction device.

A pressure characteristic curve of the pressure value changing over time is generated.

A second aspect of the embodiments of the present disclosure provides an arterial stiffness display system that is realized based on an arterial stiffness display apparatus. The arterial stiffness display apparatus includes an arm belt, a pressure sensor and a human-machine interaction device. The arterial stiffness display system includes:
a first acquisition module, used for acquiring a pressure value of the pressure sensor in a pressure rise or drop process of the arm belt;
a second acquisition module, used for generating a pressure characteristic curve of the pressure value changing over time;
a third acquisition module, used for acquiring an arterial stiffness index according to a characteristic of a curve segment, corresponding to a preset pressure value, in the pressure characteristic curve;
a fourth acquisition module, used for acquiring blood pressure values and a pulse number according to the pressure value, the blood pressure values including a highest blood pressure value and a lowest blood pressure value; and
a display module, used for displaying at most two of the blood pressure values and the pulse number, and the arterial stiffness index by means of the human-machine interaction device.

A pressure characteristic curve of the pressure value changing over time is generated.

A third aspect of the embodiments of the present disclosure provides an arterial stiffness display apparatus, including a control portion, a pump, an exhaust valve, an arm belt, a pressure sensor, a human-machine interaction device and a communication device.

The control portion is in communication connection with the pump, the pressure sensor, the human-machine interaction device and the communication device respectively. The exhaust valve is arranged on the arm belt; the arm belt is mechanically connected to the pump, the exhaust valve and the pressure sensor respectively; and the communication device is in communication connection with a cloud server.

The control portion is used for executing the steps of the above-mentioned method.

A fourth aspect of the embodiments of the present disclosure provides a computer-readable storage medium. The computer-readable storage medium stores a computer-readable instruction. The computer-readable instruction realizes, when executed by a processor, the steps of the above-mentioned method.

By means of the arterial stiffness display apparatus including the arm belt, the pressure sensor and the human-machine interaction device, the embodiments of the present disclosure acquires the pressure value applied by an artery to the pressure sensor in the pressure rise or drop process of the arm belt, acquires the pressure characteristic curve of the pressure value changing over time, and acquires, according to the characteristic of the curve segment, corresponding to the preset pressure value, in the pressure characteristic curve, the arterial stiffness index, so that the operation is simple, a relatively accurate arterial stiffness index can be obtained, and the accuracy of arterial stiffness detection can be effectively improved. The blood pressure values and the pulse number are acquired according to the pressure value, and at most two of the blood pressure values and the pulse number, and the arterial stiffness index are displayed by means of the human-machine interaction device, so that various parameters can be simultaneously measured and displayed, and the embodiments are suitable for widespread popularization and use.

It can be understood that the beneficial effects of the above second to fourth aspects can refer to related descriptions in the first aspect, and repeated descriptions are omitted here.

### Beneficial effects of the invention

### Brief explanation of the drawings

### BRIEF DESCRIPTION OF THE DRAWINGS

To describe the technical solutions in the embodiments of the present disclosure more clearly, drawings required to be used in the embodiments or the illustration of the existing art will be briefly introduced below. Obviously, the drawings in the illustration below are only some embodiments of the present disclosure. Those of ordinary skill in the art also can acquire other drawings according to the provided drawings without doing creative work.
Fig. 1 is a flow diagram of an arterial stiffness display method provided by Embodiment I of the present disclosure;
Fig. 2 to Fig. 9 are schematic diagrams of a display interface provided by Embodiment I of the present disclosure;
Fig. 10 is a schematic structural diagram of an arterial stiffness display system provided by Embodiment II of the present disclosure;
Fig. 11 to Fig. 13 are schematic structural diagrams of an arterial stiffness display apparatus provided by Embodiment III of the present disclosure.

### DESCRIPTION OF THE EMBODIMENTS

### Embodiments of the invention

In order to make those skilled in the art better understand the solutions of the present disclosure, the technical solutions in the embodiments of the present disclosure will be described clearly below in combination with the drawings in the embodiments of the present disclosure. Obviously, the embodiments described herein are only part of the embodiments of the present disclosure, not all the embodiments. Based on the embodiments in the present disclosure, all other embodiments obtained by those of ordinary skill in the art without creative work shall fall within the protection scope of the present disclosure.

The term "including" and any variations thereof in the specification and claims of the present disclosure and the above-mentioned drawings are intended to cover non-exclusive inclusions. In addition, the terms such as "first", "second", and "third" are used to distinguish different objects, rather than describing a specific order.

### Embodiment I

The present embodiment provides an arterial stiffness display method which is realized based on an arterial stiffness display apparatus. The arterial stiffness display apparatus includes a control portion, a pump, an exhaust valve, an arm belt, a pressure sensor, a temperature sensor, a humidity sensor, a barometer, a human-machine interaction device, a communication device, a motion detection device and an arm belt position detection device. The control portion includes an arm belt pressure control portion for controlling an operation speed of the pump, and a data processing portion having a data processing function. The exhaust valve is arranged on the arm belt.

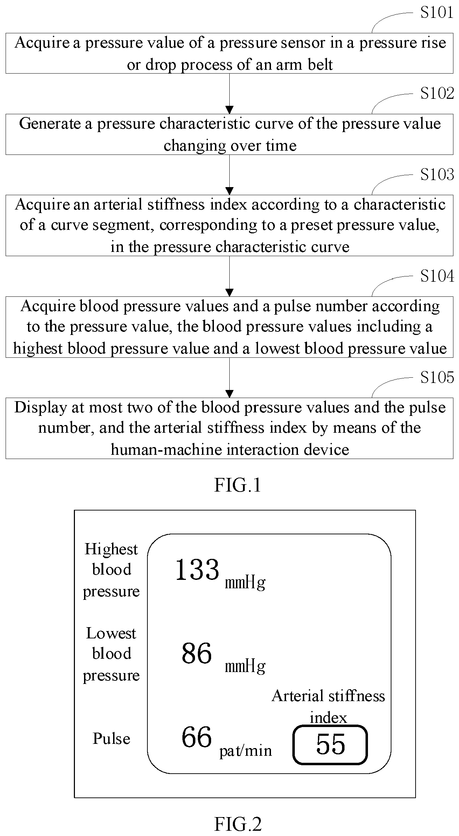

As shown in Fig. 1, the arterial stiffness display method provided by the present embodiment includes the following operations executed by the control portion:
At step S101, a pressure value of the pressure sensor is acquired in a pressure rise or drop process of the arm belt.
At step S102, a pressure characteristic curve of the pressure value changing over time is generated.

In a specific application, the amplitude of an artery usually changes regularly in a vibration process of the artery. Correspondingly, the pressure value detected by the pressure sensor would also correspondingly change, and the pressure characteristic curve of the pressure value changing over time is acquired, which is favorable for more intuitively observing the vibration of the artery, thereby effectively evaluating the arterial stiffness.

In one embodiment, before the step S102, the method includes that:
At step S100, in the pressure rise process of the arm belt, a pulse width modulation signal is output according to the pressure value of the current moment to control a rotating speed of the pump or a valve opening/closing degree of the exhaust valve to supplement and correct an air intake speed of the arm belt, so as to supplement and correct the pressure value of a next moment.

In a specific application, the data processing portion generates the pulse width modulation signal according to the pressure value, and then the arm belt pressure control portion controls the rotating speed of the pump or the valve opening/closing degree of the exhaust valve according to the pulse width modulation signal, so as to realize control of the air intake speed of the arm belt.

In a specific application, by means of supplementing and correcting the air intake speed of the current moment to supplement and correct the pressure value of the next moment, the pressure value measured by the pressure sensor can be caused to change more gently over time, thereby making the pressure characteristic curve smoother.

In one embodiment, the step S102 includes that:
At step S1021, by taking the pressure value as a dependent variable, the pressure value that changes over time is subjected to curve fitting to obtain the pressure characteristic curve of the pressure value changing over time.

In a specific application, the least square method can be used to perform the curve fitting to obtain the pressure characteristic curve.

At step S103, an arterial stiffness index is acquired according to a characteristic of a curve segment, corresponding to a preset pressure value, in the pressure characteristic curve.

In a specific application, a maximum upper limit for valuing of the preset pressure value is a maximum pressure value detected by the pressure sensor, and a lower limit for valuing is a minimum pressure value detected by the pressure sensor. The preset pressure value can be set to be any value between the minimum pressure value and the maximum pressure value according to an actual requirement.

In one embodiment, the step S103 includes that:
At step S1031, the arterial stiffness index is acquired according to a constant A and a first coefficient. The first coefficient is a coefficient related to a time duration corresponding to the preset pressure value.

In one embodiment, an expression of the step S1031 is as follows:
Arterial stiffness index=A×W;
where W denotes the first coefficient.

In one embodiment, the step S103 includes that:
At step S1032, the arterial stiffness index is acquired according to a difference between the preset pressure value and a peak value of the pressure characteristic curve, the time duration corresponding to the preset pressure value, and a second coefficient. The second coefficient is a constant related to a blood pressure.

In one embodiment, an expression of the step S1032 is as follows:
Arterial stiffness index=K1×L/H;
where K1 denotes the second coefficient; L denotes the time duration corresponding to the preset pressure value; and H denotes the difference between the preset pressure value and the peak value of the pressure characteristic curve.

In one embodiment, the step S103 includes that:
At S1033, the arterial stiffness index is acquired according to a first time duration corresponding to a first curve segment in the pressure characteristic curve, a second time duration corresponding to a second curve segment, and a third coefficient. The first curve segment is a curve segment between a wave crest in the pressure characteristic curve and a first point at the preset pressure value, and the second curve segment is a curve segment between a wave crest in the pressure characteristic curve and a second point at the preset pressure value.

In a specific application, the third coefficient is a constant related to the blood pressure, and the second coefficient and the third coefficient may be the same.

In one embodiment, an expression of the step S1033 is as follows:
Arterial stiffness index=K2×M/N;
where K2 denotes the third coefficient, M denotes the second time duration, and N denotes the first time duration.

In one embodiment, the step S103 includes that:
At S1034, the arterial stiffness index is acquired according to a fourth coefficient and a radius of an inscribed circle of a preset graph or a length of a long axis of an inscribed ellipse. The preset graph is a graph composed of a connecting line between a first point and a second point at the preset pressure value in the pressure characteristic curve and the curve segment corresponding to the preset pressure value.

In a specific application, the fourth coefficient is a constant related to the blood pressure.

In one embodiment, an expression of the step S1034 is as follows:
Arterial stiffness index=K3×R;
where K3 denotes the fourth coefficient, and R denotes the radius of the inscribed circle of the preset graph or the length of the long axis of the inscribed ellipse.

In one embodiment, the step S103 includes that:
At step S1035, the arterial stiffness index is acquired according to a fifth coefficient and a time duration corresponding to the preset pressure value.

In a specific application, the fifth coefficient is related to a ratio of the arterial stiffness index to the preset pressure value. A plurality of ratios of the arterial stiffness index to the preset pressure value are obtained in advance by means of a lot of actual measurement operations, and the fifth coefficient is obtained by taking a median, a mode or a mean of the plurality of ratios.

In one embodiment, an expression of the step S1035 is as follows:
Arterial stiffness index=K4×L;
where K4 denotes the fifth coefficient, and L denotes the time duration corresponding to the preset pressure value.

In a specific application, the unit of the arterial stiffness index obtained by means of each of the above-mentioned expressions and a standard for measuring the arteriosclerosis degree are not the same. For example, for the arterial stiffness index calculated according to any expression, the median, mode or mean of the plurality of arterial stiffness indexes obtained during calculation of the arterial stiffness indexes of a large number of users with normal vascular stiffness by means of the expression shall be taken as a standard value. If the arterial stiffness index is apparently greater than the standard value, it is indicated that the arteriosclerosis degree is large; if the arterial stiffness index is apparently less than the standard value, it is indicated that the arteriosclerosis degree is small; and if the arterial stiffness index is close or equal to the standard value, it is indicated that the arteriosclerosis degree is normal.

In one embodiment, after the step S101, the method includes that:
At step S201, a plurality of arterial vibration amplitude curves of an arterial vibration amplitude of a user changing over time are acquired according to the pressure value;
at step 202, the arterial stiffness index is acquired according to a sixth coefficient, and an arterial vibration amplitude maximum peak value and an arterial vibration amplitude minimum peak value in the plurality of arterial vibration amplitude curves.

In a specific application, the arterial vibration amplitude of the user is in direct proportion to a pressure value of the pressure sensor; and the arterial vibration amplitude of the user can be obtained by means of detecting the pressure value and performing data conversion, thereby obtaining the arterial vibration amplitude curves where the arterial vibration amplitude of the user changes over time. Since an artery vibrates periodically, the arterial vibration amplitude also changes periodically. Therefore, the plurality of arterial vibration amplitude curves can be obtained within a plurality of periods of time.

In one embodiment, an expression of the step S202 is as follows:
Arterial stiffness index=K5×∑Hi/∑Li;
where K5 denotes the sixth coefficient, Hi denotes the arterial vibration amplitude maximum peak value in the arterial vibration amplitude curves, Li denotes the arterial vibration amplitude minimum peak value in the arterial vibration amplitude curves, a value range of i is [1, n], and i and n are integers.

In a specific application, the sixth coefficient is a constant related to the blood pressure. n can be any integer value needing to be set to be greater than 1 according to an actual requirement. If the value of n is larger, the arterial stiffness index is more accurate. In case of n=5, the arterial stiffness index=K5×(H1 +H2 +H3 +H4 +H5)/(L1 +L2 +L3 +L4 +L5). That is, the arterial stiffness index is obtained by multiplying a ratio of a sum of the maximum peak values to the minimum peak values of five arterial vibration amplitude curves in the plurality of arterial vibration amplitude curves by the sixth coefficient.

In one embodiment, after the step S102, the method includes that:
A temperature value of the temperature sensor, a humidity value of the humidity sensor, an air pressure value of the barometer, and body information and unhealthy living habit information input by the user by means of the human-machine interaction device are acquired; and the pressure characteristic curve is corrected according to the temperature value, the humidity value, the air pressure value, the body information and the unhealthy living habit information.

In a specific application, the body information includes blood pressure values, the gender, the age, the height, the body weight, past medical history and other information of the user. The unhealthy living habit information includes biochemical habit information such as smoking, bibulosity and staying up late. The different pressure characteristic curves acquired during measurement of the arterial stiffness indexes of different users are corrected in a targeted manner according to the body information and the unhealthy living habit information of the different users. Correction solutions for the different users are set by means of a large amount of actually measured data in advance.

In one embodiment, the step S101 includes that:
At step S1011, in the pressure rise process of the arm belt, a motion range of the user is detected by means of the motion detection device, and a wear position of the arm belt is detected by means of the arm belt position detection device.
At step S1012, when the motion range is less than or equal to a preset range and the wear position is correct, the pressure value of the pressure sensor is acquired.
At step S1013, when the motion range is greater than the preset range and the wear position is wrong, the pressure value is deleted, and the human-machine interaction device sends an error prompt.

In a specific application, the wear position of the arm belt and the motion range of the user directly affect the accuracy of a measurement result of the arterial stiffness index. When the user wears the arm belt improperly, or has a large motion range, the pressure value acquired by the pressure sensor is inaccurate, and cannot be used to obtain an accurate arterial stiffness index, so that the pressure value needs to be deleted, and a prompt is sent that the wear position of the arm belt on the user is wrong or an action is wrong. Theoretically, the user should wear the arm belt at the upper arm, and keep the body in a static state with smooth breathing as much as possible.

At step S104, blood pressure values and a pulse number are acquired according to the pressure value, the blood pressure values including a highest blood pressure value (systolic blood pressure) and a lowest blood pressure value (diastolic blood pressure).

In a specific application, the blood pressure values and the pulse number can be obtained by performing conversion processing on the pressure value. A pulse pressure value and a cardiac function index of the user can also be acquired according to the pressure value.

At step S105, at most two of the blood pressure values and the pulse number, and the arterial stiffness index are displayed by means of the human-machine interaction device.

In a specific application, the human-machine interaction device is used for displaying the arterial stiffness index, and can also selectively display the blood pressure values and/or the pulse number according to an actual requirement. As shown in Fig. 2, it exemplarily illustrates an arterial stiffness index, a highest blood pressure value, a lowest blood pressure value and a pulse number which are simultaneously displayed in a digital form.

In one embodiment, the arterial stiffness display method further includes that:
whether the arterial stiffness index is greater than a preset arterial stiffness index threshold is determined; when the arterial stiffness index is greater than the preset arterial stiffness index threshold, a prompt that the arterial stiffness index exceeds the standard is sent by means of the human-machine interaction device; or whether the highest blood pressure value is greater than a preset blood pressure upper limit threshold is determined, and whether the lowest blood pressure value is less than a preset blood pressure lower limit value is determined; when the highest blood pressure value is greater than the preset blood pressure upper limit threshold or the lowest blood pressure value is less than the preset blood pressure lower limit value, a prompt that the blood pressure value exceeds the standard is sent by means of the human-machine interaction device; or whether the pulse number is in a preset pulse number range is determined; and when the pulse number is not in the preset pulse number range, a prompt that the pulse number exceeds the standard is sent by means of the human-machine interaction device.

In a specific application, the arterial stiffness index, the blood pressure values and the pulse each have a normal range representing that the user is healthy. When a certain indicator exceeds the normal range, it is represented that the health state of the user is abnormal, and corresponding prompts need to be thus sent to warn the user. A specific prompt mode may be to send the prompt by means of a pop-up window, a warning mark or a voice prompt. When the prompt mode is to display the warning mark, the warning mark can be displayed in a display region where a corresponding overproof parameter.

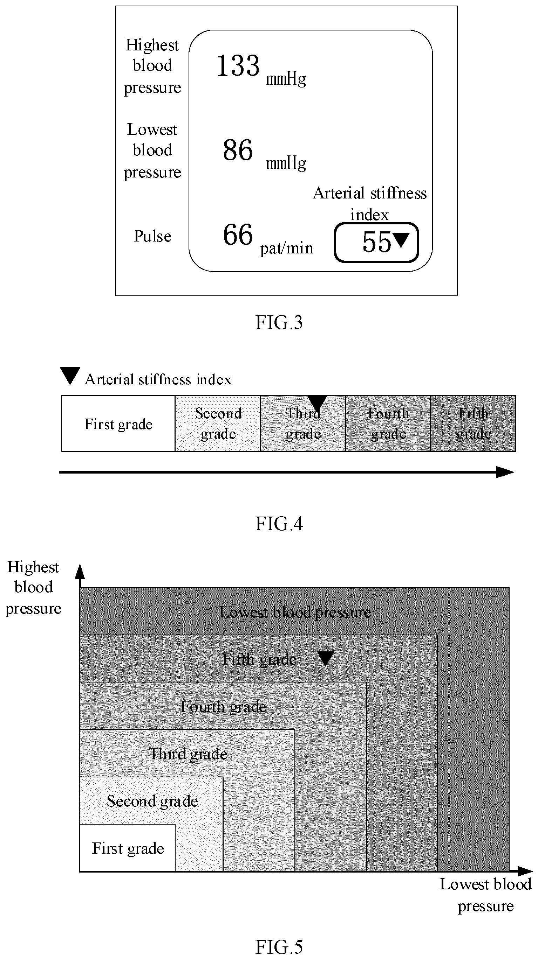

As shown in Fig. 3, it exemplarily illustrates a warning mark displayed in an arterial stiffness index display region when the arterial stiffness index is greater than the preset arterial stiffness index threshold.

In one embodiment, the arterial stiffness display method further includes that: an arteriosclerosis degree of the user is determined according to the arterial stiffness index; the arteriosclerosis degree is displayed by means of the human-machine interaction device; the hypertension degree of the user is determined according to the blood pressure values; and the hypertension degree is displayed by means of the human-machine interaction device.

In a specific application, different arteriosclerosis degrees and/or different hypertension degrees are distinguished and displayed in one or more forms of character, graph, sheet and different colors.

As shown in Fig. 4, it exemplarily illustrates different arteriosclerosis degrees are distinguished and displayed simultaneously by means of characters, graphs and different colors. The arteriosclerosis degree includes five grades that indicate gradual change from normal to severe. There is an inverted triangle warning mark displayed in the third grade if the arterial stiffness index falls into the third grade.

In one embodiment, the arterial stiffness display method further includes: the blood pressure values and the hypertension degree are displayed in the form of a two-dimensional coordinate graph by means of the human-machine interaction device. In a specific application, the two-dimensional coordinate graph can include one or more display elements such as characters, graphs, sheets and different colors to distinguish and display different blood pressure values and different hypertension degrees.

As shown in Fig. 5, it exemplarily illustrates a two-dimensional coordinate graph where different hypertension degrees are distinguished and displayed in different colors. The horizontal coordinate indicates the lowest blood pressure value, and the vertical coordinate indicates the highest blood pressure value. The dot in the two-dimensional coordinate graph indicates a coordinate position of a blood pressure value. The hypertension degree includes six grades that indicate gradual change from normal to severe. There is an inverted triangle warning mark displayed in the fifth grade if the blood pressure value falls into the fifth grade.

In one embodiment, the arterial stiffness display method further includes that:
the arteriosclerosis degree and the hypertension degree are displayed in the form of a two-dimensional coordinate graph by means of the human-machine interaction device. In a specific application, the two-dimensional coordinate graph can also be used for distinguishing and displaying different arteriosclerosis degrees and different hypertension degrees.

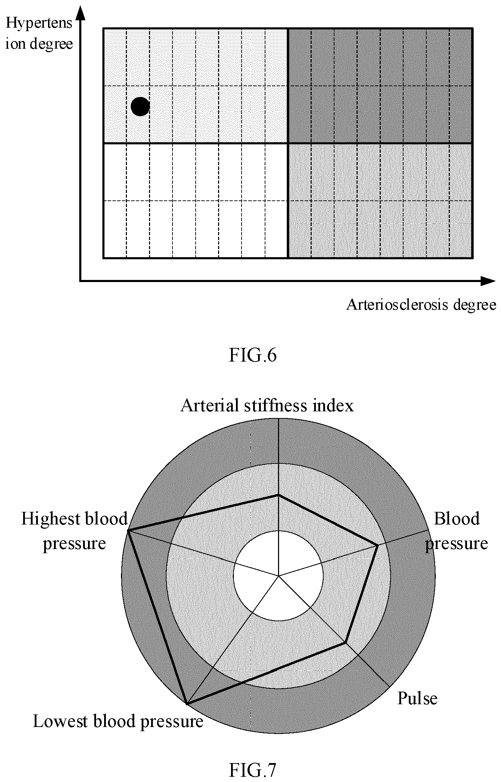

As shown in Fig. 6, it exemplarily illustrates a two-dimensional coordinate graph, the horizontal coordinate of which indicates the arteriosclerosis degree, and the vertical coordinate of which indicates the hypertension degree. The dot in the two-dimensional coordinate graph falls in a region that represents the hypertension is severe and the arterial stiffness is normal.

In one embodiment, the step S105 includes that:
at most two of the arteriosclerosis degree and the hypertension degree, and the arterial stiffness index, the blood pressure values and the pulse number are displayed in a form of a multidimensional tendency graph by means of the human-machine interaction device.

In a specific application, a plurality of parameters in the arteriosclerosis degree, the hypertension degree, the arterial stiffness index, the blood pressure values and the pulse number can be simultaneously displayed by means of the multidimensional tendency graph. In a specific application, the multidimensional tendency graph may include one or more display elements such as characters, graphs, sheets and different colors.

As shown in Fig. 7, it exemplarily illustrates a five-dimensional tendency graph used for simultaneously displaying the arteriosclerosis degree, the hypertension degree, the arterial stiffness index, the blood pressure values and the pulse number. Each parameter includes three grades from normal to severe. The three grades are distinguished and displayed by circular or circular-ring regions in different colors in the five-dimensional tendency graph. The arteriosclerosis degree, the hypertension degree, the arterial stiffness index, the blood pressure values and the pulse number of the user are connected by means of straight lines to form a pentagon. If a parameter is closer to a central region of the five-dimensional tendency graph, it represents that the severity is lower or the numerical value is smaller; on the contrary, if a parameter is closer to an edge region of the five-dimensional tendency graph, it represents that the severity is higher or the numerical value is larger.

In one embodiment, the arterial stiffness display method further includes that: at most four of the blood pressure values, the pulse number, the arteriosclerosis degree and the hypertension degree, and the arterial stiffness index are displayed in a form of a dial gauge by means of the human-machine interaction device.

In a specific application the dial gauge can be specifically displayed in an arc-shaped or round plate form. The dial gauge may include one or more display elements such as characters, graphs and different colors to distinguish and display different blood pressure values, pulse numbers, arteriosclerosis degrees, hypertension degrees, arterial stiffness indexes and the like.

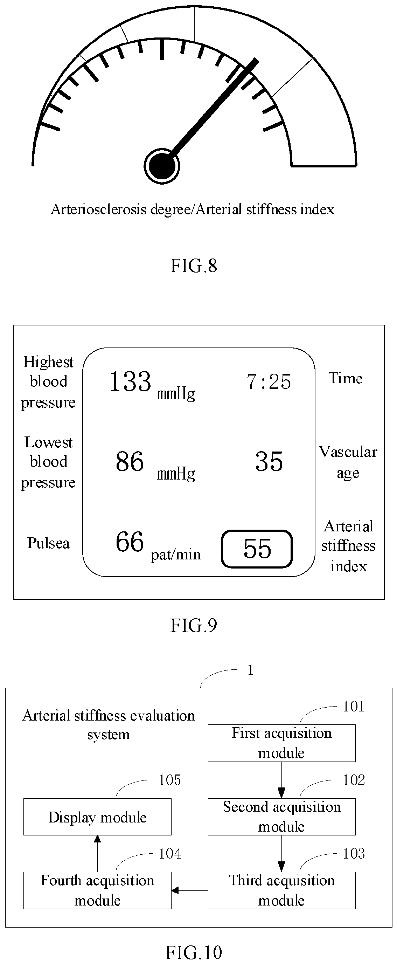

As shown in Fig. 8, it exemplarily illustrates a dial gauge used for simultaneously displaying the arteriosclerosis degree and the arterial stiffness index. The arteriosclerosis degree includes 5 scales and 6 scale ranges from normal to severe; the dial gauge is displayed in the arc-shaped plate form; a pointer in the plate is used for pointing a scale corresponding to a numerical value of the arterial stiffness index and a scale or scale range corresponding to the arteriosclerosis degree. If the arteriosclerosis degree and the arterial stiffness index of the user are larger, the pointer is closer to the right side; on the contrary, if the arteriosclerosis degree and the arterial stiffness index of the user are smaller, the pointer is closer to the left side.

In one embodiment, the arterial stiffness display method further includes that: current time is acquired; the current time is displayed by means of the human-machine interaction device; the vascular age of the user is analyzed according to the arterial stiffness index; the vascular age is displayed by means of the human-machine interaction device; a curve or histogram of at least one of the arterial stiffness index, the blood pressure value, the pulse number and the vascular age that changes over time is acquired; and the curve or histogram is displayed in a form of a two-dimensional coordinate graph by means of the human-machine interaction device.

In a specific application, the current time can also be displayed, and the vascular age of the user is obtained by analysis according to the arterial stiffness index. Furthermore, changes over time of the arterial stiffness index, the blood pressure values, the pulse number or the vascular age can be displayed in the form of a curve or histogram in chronological order. When the curve or histogram is displayed in a form of a two-dimensional coordinate graph, the horizontal coordinate indicates time, and the vertical coordinate indicates the arterial stiffness index, the blood pressure value, the pulse number or the vascular age.

As shown in Fig. 9, it exemplarily illustrates an arterial stiffness index, a blood pressure value, a pulse number, a vascular age and the time which are simultaneously displayed in a numerical form.

In one embodiment, the arterial stiffness display method further includes that: a display mode switching instruction input by the user by means of the human-machine interaction device is acquired; and the display mode of the human-machine interaction device is switched according to the display mode switching instruction.

In a specific application, the user can input the display mode switching instruction by means of the human-machine interaction device according to an actual requirement, so as to display all the parameters in different display modes. Specific display modes include, but are not limited to, the display modes in the embodiments corresponding to Fig. 10 to Fig. 17.

In one embodiment, the arterial stiffness display method further includes that: the risk at which the user suffers from a cardiovascular disease is analyzed according to the arterial stiffness index, the blood pressure values and the pulse number, and an analysis result is generated; and the analysis result is displayed by means of the human-machine interaction device.

In a specific application, the arterial stiffness display apparatus specifically compares the measured arterial stiffness index, blood pressure values and pulse number of the user with thresholds corresponding to all the parameters. If the parameters are greater than the thresholds, it is indicated that there is a risk of a corresponding disease; and on the contrary, it is indicated that there is no risk. An analysis standard of the cardiovascular disease can be preset and stored, and analysis is performed based on this analysis standard. The analysis result can be displayed in various forms such as characters, graphs and sheets, and a voice prompt can also be available.

In one embodiment, the arterial stiffness display method further includes that: the arterial stiffness index, the blood pressure values, the pulse number and the analysis result are sent to a cloud server by means of the communication device, the cloud server being used for sending the arterial stiffness index, the blood pressure values, the pulse number and the analysis result to a client of a doctor, and the doctor diagnosing a disease of the user, and generating a disease diagnosis result and sending the same to the cloud server by means of the client; and the disease diagnosis result sent by the cloud server is received by means of the communication device; and the disease diagnosis result is displayed by means of the human-machine interaction device.

By means of the arterial stiffness display apparatus including the arm belt, the pressure sensor and the human-machine interaction device, the present embodiment acquires the pressure value applied by an artery to the pressure sensor in the pressure rise or drop process of the arm belt, acquires the pressure characteristic curve of the pressure value changing over time, and acquires, according to the characteristic of the curve segment, corresponding to the preset pressure value, in the pressure characteristic curve, the arterial stiffness index, so that the operation is simple, a relatively accurate arterial stiffness index can be obtained, and the accuracy of arterial stiffness detection can be effectively improved. The blood pressure values and the pulse number are acquired according to the pressure value, and at most two of the blood pressure values and the pulse number, and the arterial stiffness index are displayed by means of the human-machine interaction device, so that various parameters can be simultaneously measured and displayed, and the present embodiment is suitable for widespread popularization and use.

It should be understood that the serial numbers of all the steps in the above embodiment do not mean the order of execution. The order of execution of the all processes shall be determined by their functions and an internal logic, and shall not constitute any limitations to the implementation processes of the embodiments of the present disclosure.

### Embodiment II

As shown in Fig. 10, the present embodiment provides an arterial stiffness display system 1 used for executing the steps of the method in Embodiment I. The arterial stiffness display system 1 is realized based on the arterial stiffness display apparatus in Embodiment I, and may be specifically a software program system in the control portion. The arterial stiffness display system 1 includes:
a first acquisition module 101, used for acquiring a pressure value of the pressure sensor in a pressure rise or drop process of the arm belt;
a second acquisition module 102, used for generating a pressure characteristic curve of the pressure value changing over time;
a third acquisition module 103, used for acquiring an arterial stiffness index according to a characteristic of a curve segment, corresponding to a preset pressure value, in the pressure characteristic curve;
a fourth acquisition module 104, used for acquiring blood pressure values and a pulse number according to the pressure value, the blood pressure values including a highest blood pressure value and a lowest blood pressure value; and
a display module 105, used for displaying at most two of the blood pressure values and the pulse number and the arterial stiffness index by means of the human-machine interaction device. In one embodiment, the arterial stiffness display system further includes:
a fifth acquisition module, used for acquiring, according to the pressure value, a plurality of arterial vibration amplitude curves of an arterial vibration amplitude of a user changing over time;
a sixth acquisition module, used for acquiring the arterial stiffness index according to a sixth coefficient, and an arterial vibration amplitude maximum peak value and an arterial vibration amplitude minimum peak value in the plurality of arterial vibration amplitude curves.

In one embodiment, the arterial stiffness display system further includes:
a seventh acquisition module, used for acquiring a temperature value of the temperature sensor, a humidity value of the humidity sensor, an air pressure value of the barometer, and body information and unhealthy living habit information input by the user by means of the human-machine interaction device;
a correction module, used for correcting the pressure characteristic curve according to the temperature value, the humidity value, the air pressure value, the body information and the unhealthy living habit information.

In one embodiment, the arterial stiffness display system further includes:
a first judgment module, used for determining whether the arterial stiffness index is greater than a preset arterial stiffness index threshold;
a first prompt module, used for sending a prompt that the arterial stiffness index exceeds the standard by means of the human-machine interaction device when the arterial stiffness index is greater than the preset arterial stiffness index threshold;
a second judgment module, used for determining whether the highest blood pressure value is greater than a preset blood pressure upper limit threshold is determined, and whether the lowest blood pressure value is less than a preset blood pressure lower limit value;
a second prompt module, used for sending a prompt that the blood pressure value exceeds the standard by means of the human-machine interaction device when the highest blood pressure value is greater than the preset blood pressure upper limit threshold or the lowest blood pressure value is less than the preset blood pressure lower limit value;
a third judgment module, used for determining whether the pulse number is in a preset pulse number range;
a third prompt module, used for sending a prompt that the pulse number exceeds the standard by means of the human-machine interaction device when the pulse number is not in the preset pulse number range.

In one embodiment, the arterial stiffness display system further includes:
a first determination module, used for determining an arteriosclerosis degree of the user according to the arterial stiffness index, the display module being also used for displaying the arteriosclerosis degree by means of the human-machine interaction device;
a second determination module, used for determining a hypertension degree of the user according to the blood pressure values, the display module being also used for displaying the hypertension degree by means of the human-machine interaction device.

In one embodiment, the display module is also used for: displaying the blood pressure values and the hypertension degree in a form of a two-dimensional coordinate graph by means of the human-machine interaction device; displaying the arteriosclerosis degree and the hypertension degree in a form of a two-dimensional coordinate graph by means of the human-machine interaction device; displaying at most two of the arteriosclerosis degree and the hypertension degree, and the arterial stiffness index, the blood pressure values and the pulse number in a form of a multidimensional tendency graph by means of the human-machine interaction device; and displaying at most four of the blood pressure values, the pulse number, the arteriosclerosis degree and the hypertension degree, and the arterial stiffness index in a form of a dial gauge by means of the human-machine interaction device

In one embodiment, the arterial stiffness display system further includes:

an eighth acquisition module, used for acquiring current time, the display module being also used for displaying the current time by means of the human-machine interaction device; an analysis module, used for analyzing the vascular age of the user according to the arterial stiffness index, the display module being also used for displaying the vascular age by means of the human-machine interaction device; and a generation module, used for generating a curve or histogram of at least one of the arterial stiffness index, the blood pressure value, the pulse number and the vascular age that changes over time, and
the display module being also used for displaying the curve or histogram in a form of a two-dimensional coordinate graph by means of the human-machine interaction device.

In one embodiment, the arterial stiffness display system further includes:
a ninth acquisition module, used for acquiring a display mode switching instruction input by the user by means of the human-machine interaction device;
a switching module, used for switching a display mode of the human-machine interaction device according to the display mode switching instruction.

In one embodiment, the analysis module is also used for analyzing the risk, at which the user suffers from a cardiovascular disease, according to the arterial stiffness index, the blood pressure values and the pulse number, and generating an analysis result.

The display module is also used for displaying the analysis result by means of the human-machine interaction device.

In one embodiment, the arterial stiffness display system further includes:
a communication module, used for sending the arterial stiffness index, the blood pressure values, the pulse number and the analysis result to a cloud server by means of the communication device, the cloud server being used for sending the arterial stiffness index, the blood pressure values, the pulse number and the analysis result to a client of a doctor, and the doctor diagnosing a disease of the user, and generating a disease diagnosis result and sending the same to the cloud server by means of the client.

The communication module is also used for receiving the disease diagnosis result sent by the cloud server by means of the communication device.

The display module is also used for displaying the disease diagnosis result by means of the human-machine interaction device.

In a specific application, all the above-mentioned modules can be realized by means of mutually independent processors, or can be commonly integrated into one processor.

By means of the arterial stiffness display apparatus including the arm belt, the pressure sensor and the human-machine interaction device, the present embodiment acquires the pressure value applied by an artery to the pressure sensor in the pressure rise or drop process of the arm belt, acquires the pressure characteristic curve of the pressure value changing over time, and acquires, according to the characteristic of the curve segment, corresponding to the preset pressure value, in the pressure characteristic curve, the arterial stiffness index, so that the operation is simple, a relatively accurate arterial stiffness index can be obtained, and the accuracy of arterial stiffness detection can be effectively improved. The blood pressure values and the pulse number are acquired according to the pressure value, and at most two of the blood pressure values and the pulse number, and the arterial stiffness index are displayed by means of the human-machine interaction device, so that various parameters can be simultaneously measured and displayed, and the present embodiment is suitable for widespread popularization and use.

### Embodiment III

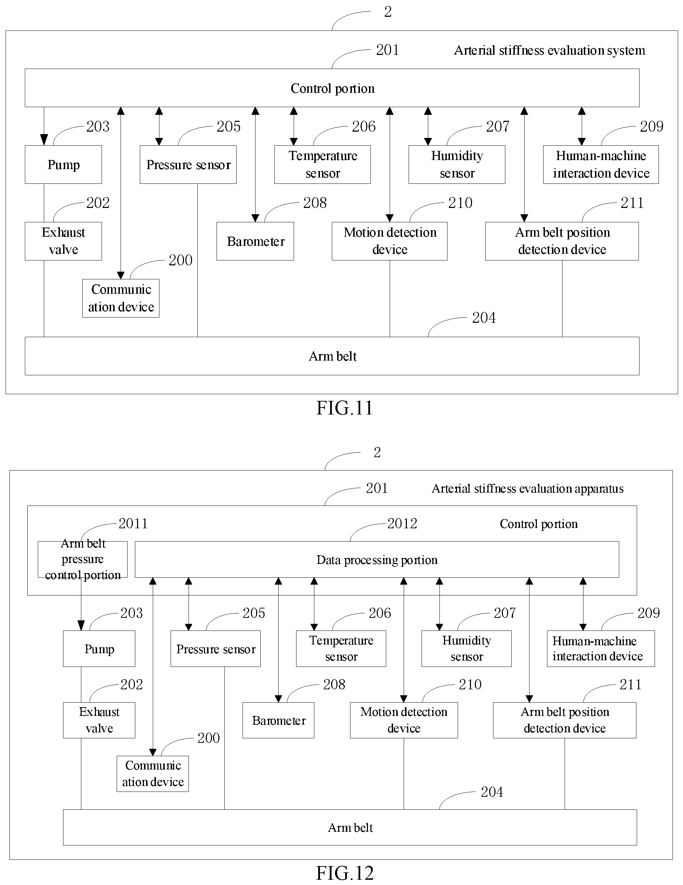

As shown in Fig. 11, the present embodiment provides an arterial stiffness display apparatus 2, including: a control portion 201, an exhaust valve 202, a pump 203, an arm belt 204, a pressure sensor 205, a temperature sensor 206, a humidity sensor 207, a barometer 208, a human-machine interaction device 209, a motion detection device 210, an arm belt position detection device 211 and a communication device 200.

The control portion 201 is in communication connection with the pump 203, the pressure sensor 205, the temperature sensor 206, the humidity sensor 207, the barometer 208, the human-machine interaction device 209, the motion detection device 210, the arm belt position detection device 211 and the communication device 200; the exhaust valve 202 is arranged on the arm belt 204; the arm belt 204 is mechanically connected to the pump 203, the exhaust valve 202, the pressure sensor 205, the motion detection device 210 and the arm belt position detection device 211 respectively; and the communication device 200 is in communication connection with a cloud server 3; and the cloud server 3 is in communication connection with a client 4 of a doctor.

The control portion 201 is used for executing the steps of the arterial stiffness display method in Embodiment I.

As shown in Fig. 12, in one embodiment, the control portion 201 includes an arm belt pressure control portion 2011 and a data processing portion 2012; the arm belt pressure control portion 2011 is in communication connection with the data processing portion 2012 and the pump 203; and the data processing portion 2012 is in communication connection with the pressure sensor 205, the temperature sensor 206, the humidity sensor 207, the barometer 208, the human-machine interaction device 209, the motion detection device 210, the arm belt position detection device 211 and the communication device 200.

The arm belt pressure control portion 2011 and the data processing portion 2012 are used for executing the steps of the arterial stiffness display method in Embodiment I.

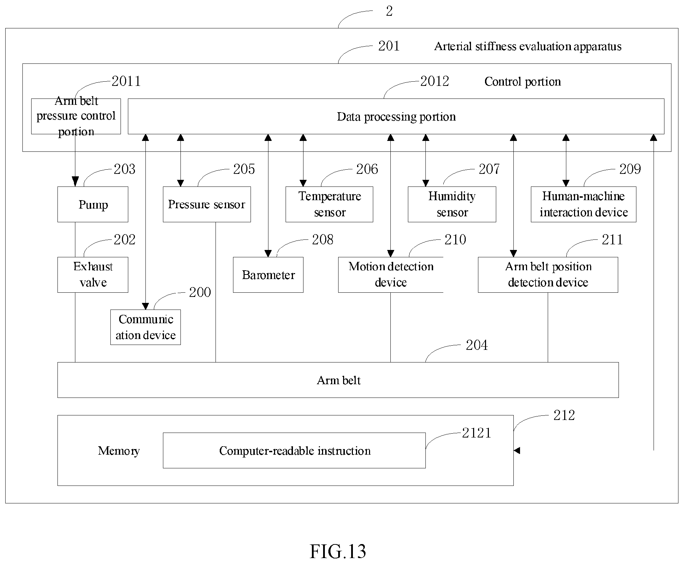

As shown in Fig. 13, in one embodiment, both the arm belt pressure control portion 2011 and the data processing portion 2012 can be mutually independent processors or commonly integrated into the same processor. The arterial stiffness display apparatus 2 further includes a memory 212, and a computer-readable instruction 2121 which is stored in the memory 212 and can be operated on the processor. The processor executes the computer-readable instruction to realize the steps of the method in Embodiment I or the functions of all the modules in Embodiment II.

The arterial stiffness display apparatus 2 may be an electronic sphygmomanometer. The arterial stiffness display apparatus 2 may include, but not limited to, a processor and a memory 212. Those skilled in the art can understand that Fig. 10 is merely illustrative of the arterial stiffness display apparatus 2, and does not constitute a limitation to the arterial stiffness display apparatus 2.

The memory 212 may be an internal storage unit of the arterial stiffness display apparatus 2. The memory 212 may also be an external storage device of the arterial stiffness display apparatus 2, such as a plug-in hard disk provided on the arterial stiffness display apparatus 2. Further, the memory 212 may also include both the internal storage unit and the external storage unit of the arterial stiffness display apparatus 2. The memory 212 is used for storing the computer-readable instruction and other readable instructions and data required by the arterial stiffness display apparatus 2. The memory 212 may also be used for temporarily storing data that has been output or is to output.

Those skilled in the art can clearly understand that, for convenience and conciseness of description, only the division of the above-mentioned functional units and modules is used for illustration. In addition, specific names of all the functional units and modules are only for the convenience of distinguishing each other, and are not used to limit the protection scope of the present disclosure. For the specific working process of the units and modules in the foregoing system, reference may be made to the corresponding process in the foregoing method embodiment, which is not repeated here.

Those skilled in the art may realize that the units and algorithm steps of all the examples described in combination with the embodiments disclosed herein can be implemented by electronic hardware or a combination of computer software and electronic hardware. Whether these functions are executed by hardware or software depends on the specific application and design constraints of the technical solution. Professionals can use different methods for each specific application to implement the described functions, but such implementation should not be considered as going beyond the scope of the present disclosure.

In the embodiments provided by the present disclosure, it should be understood that the disclosed apparatus/terminal device and method may be implemented in other ways. The units described as separate components may or may not be physically separated, and the components displayed as units may or may not be physical units, that is, they may be located in one place, or they may be distributed on multiple network units. Part or all of the units may be selected according to actual needs to achieve the objectives of the solutions of the embodiments.

In addition, all functional units in all the embodiments of the present disclosure can be integrated into one processing unit, or each unit can physically exist alone, or two or more units can be integrated in one unit. The above integrated units can be implemented in the form of hardware, or can be implemented in the form of software functional units.

The integrated module/unit, if implemented in the form of a software functional unit and sold or used as a standalone product, may be stored in a computer readable storage medium. Based on this understanding, the present disclosure implements all or part of the flows in the above-mentioned embodiment methods, and can also be completed by instructing relevant hardware through computer-readable instructions. The computer-readable instructions can be stored in a computer-readable storage medium. The computer-readable instruction can realize, when executed by the processor, the steps of the foregoing method embodiments. The computer-readable instructions include computer-readable instruction codes, and the computer-readable instruction codes may be in the form of source codes, object codes, executable files, or some intermediate forms. The computer-readable medium may include: any entity or apparatus capable of carrying the computer-readable instruction code, recording medium, U disk, mobile hard disk, magnetic disk, optical disk, computer memory, read-only aeaory (ROA), random access aeaory (RAA), electric carrier signal, telecommunications signal, software distribution medium, and the like. It should be noted that the content contained in the computer-readable medium can be appropriately added or deleted according to the requirements of the legislation and patent practice in the jurisdiction. For example, in some jurisdictions, according to the legislation and patent practice, the computer-readable medium does not include electrical carrier signals and telecommunication signals.

The above-mentioned embodiments are only used to illustrate the technical solutions of the present disclosure, not intended to limit them. Although the present disclosure has been described in detail with reference to the foregoing embodiments, those of ordinary skill in the art should understand that they still can modify the technical solutions recorded in all the foregoing embodiments, or replace partial technical features by equivalences. These modifications and replacements do not cause the essences of the corresponding technical solutions to depart from the spirit and scope of the technical solutions of all the embodiments of the present disclosure, and shall fall within the protection scope of the present disclosure.

## Claims

1. An arterial stiffness display method, realized based on an arterial stiffness display apparatus, wherein the arterial stiffness display apparatus comprises an arm belt, a pressure sensor and a human-machine interaction device; the arterial stiffness display method comprises:
acquiring a pressure value of the pressure sensor in a pressure rise or drop process of the arm belt;
generating a pressure characteristic curve of the pressure value changing over time;
acquiring an arterial stiffness index according to a characteristic of a curve segment, corresponding to a preset pressure value, in the pressure characteristic curve;
acquiring blood pressure values and a pulse number according to the pressure value, the blood pressure values comprising a highest blood pressure value and a lowest blood pressure value; and
displaying at most two of the blood pressure values and the pulse number, and the arterial stiffness index by means of the human-machine interaction device.

2. The arterial stiffness display method according to claim 1, further comprising:
determining whether the arterial stiffness index is greater than a preset arterial stiffness index threshold;
when the arterial stiffness index is greater than the preset arterial stiffness index threshold, sending a prompt that the arterial stiffness index exceeds the standard by means of the human-machine interaction device;
or, determining whether the highest blood pressure value is greater than a preset blood pressure upper limit threshold, and determining whether the lowest blood pressure value is less than a preset blood pressure lower limit value;
when the highest blood pressure value is greater than the preset blood pressure upper limit threshold or the lowest blood pressure value is less than the preset blood pressure lower limit value, sending a prompt that the blood pressure value exceeds the standard by means of the human-machine interaction device;
or, determining whether the pulse number is in a preset pulse number range;
when the pulse number is not in the preset pulse number range, sending a prompt that the pulse number exceeds the standard by means of the human-machine interaction device.

3. The arterial stiffness display method according to claim 1, further comprising:
determining an arteriosclerosis degree of a user according to the arterial stiffness index;
displaying the arteriosclerosis degree by means of the human-machine interaction device;
determining a hypertension degree of the user according to the blood pressure values;
displaying the hypertension degree by means of the human-machine interaction device.

4. The arterial stiffness display method according to claim 3, further comprising:
displaying the blood pressure values and the hypertension degree in a form of a two-dimensional coordinate graph by means of the human-machine interaction device;
or, displaying the arteriosclerosis degree and the hypertension degree in a form of a two-dimensional coordinate graph by means of the human-machine interaction device;
or, displaying at most two of the arteriosclerosis degree and the hypertension degree, and the arterial stiffness index, the blood pressure values and the pulse number in a form of a multidimensional tendency graph by means of the human-machine interaction device;
or, displaying at most four of the blood pressure values, the pulse number, the arteriosclerosis degree and the hypertension degree, and the arterial stiffness index in a form of a dial gauge by means of the human-machine interaction device.

5. The arterial stiffness display method according to claim 1, further comprising:
acquiring current time;
displaying the current time by means of the human-machine interaction device;
analyzing the vascular age of the user according to the arterial stiffness index;
displaying the vascular age by means of the human-machine interaction device;
generating a curve or histogram of at least one of the arterial stiffness index, the blood pressure value, the pulse number and the vascular age that changes over time;
displaying the curve or histogram in a form of a two-dimensional coordinate graph by means of the human-machine interaction device.

6. The arterial stiffness display method according to claim 1, further comprising:
acquiring a display mode switching instruction input by the user by means of the human-machine interaction device;
switching a display mode of the human-machine interaction device according to the display mode switching instruction.

7. The arterial stiffness display method according to claim 1, further comprising:
analyzing the risk, at which the user suffers from a cardiovascular disease, according to the arterial stiffness index, the blood pressure values and the pulse number, and generating an analysis result;
displaying the analysis result by means of the human-machine interaction device.

8. The arterial stiffness display method according to claim 7, wherein the arterial stiffness display apparatus further comprises a communication device, and the arterial stiffness display method further comprises:
sending the arterial stiffness index, the blood pressure values, the pulse number and the analysis result to a cloud server by means of the communication device, the cloud server being used for sending the arterial stiffness index, the blood pressure values, the pulse number and the analysis result to a client of a doctor, and the doctor diagnosing a disease of the user, and generating a disease diagnosis result and sending the same to the cloud server by means of the client;
receiving the disease diagnosis result sent by the cloud server by means of the communication device;
displaying the disease diagnosis result by means of the human-machine interaction device.

9. An arterial stiffness display system, realized based on an arterial stiffness display apparatus, wherein the arterial stiffness display apparatus comprises an arm belt, a pressure sensor and a human-machine interaction device; the arterial stiffness display system comprises:
a first acquisition module, used for acquiring a pressure value of the pressure sensor in a pressure rise or drop process of the arm belt;
a second acquisition module, used for generating a pressure characteristic curve of the pressure value changing over time;
a third acquisition module, used for acquiring an arterial stiffness index according to a characteristic of a curve segment, corresponding to a preset pressure value, in the pressure characteristic curve;
a fourth acquisition module, used for acquiring blood pressure values and a pulse number according to the pressure value, the blood pressure values comprising a highest blood pressure value and a lowest blood pressure value; and
a display module, used for displaying at most two of the blood pressure values and the pulse number, and the arterial stiffness index by means of the human-machine interaction device.

10. An arterial stiffness display apparatus, comprising a control portion, a pump, an exhaust valve, an arm belt, a pressure sensor, a human-machine interaction device and a communication device,
wherein the control portion is in communication connection with the pump, the pressure sensor, the human-machine interaction device and the communication device respectively; the exhaust valve is arranged on the arm belt; the arm belt is mechanically connected to the pump, the exhaust valve and the pressure sensor respectively; the communication device is in communication connection with a cloud server;
the control portion is used for executing the following steps:
acquiring a pressure value of the pressure sensor in a pressure rise or drop process of the arm belt;
generating a pressure characteristic curve of the pressure value changing over time;
acquiring an arterial stiffness index according to a characteristic of a curve segment, corresponding to a preset pressure value, in the pressure characteristic curve;
acquiring blood pressure values and a pulse number according to the pressure value, the blood pressure values comprising a highest blood pressure value and a lowest blood pressure value; and
displaying at most two of the blood pressure values and the pulse number, and the arterial stiffness index by means of the human-machine interaction device.

11. The arterial stiffness display apparatus according to claim 10, wherein the control portion is further used for executing the following steps:
determining whether the arterial stiffness index is greater than a preset arterial stiffness index threshold;
when the arterial stiffness index is greater than the preset arterial stiffness index threshold, sending a prompt that the arterial stiffness index exceeds the standard by means of the human-machine interaction device;
or, determining whether the highest blood pressure value is greater than a preset blood pressure upper limit threshold, and determining whether the lowest blood pressure value is less than a preset blood pressure lower limit value;
when the highest blood pressure value is greater than the preset blood pressure upper limit threshold or the lowest blood pressure value is less than the preset blood pressure lower limit value, sending a prompt that the blood pressure value exceeds the standard by means of the human-machine interaction device;
or, determining whether the pulse number is in a preset pulse number range;
when the pulse number is not in the preset pulse number range, sending a prompt that the pulse number exceeds the standard by means of the human-machine interaction device.

12. The arterial stiffness display apparatus according to claim 10, wherein the control portion is further used for executing the following steps:
determining an arteriosclerosis degree of a user according to the arterial stiffness index;
displaying the arteriosclerosis degree by means of the human-machine interaction device;
determining a hypertension degree of the user according to the blood pressure values;
displaying the hypertension degree by means of the human-machine interaction device.

13. The arterial stiffness display apparatus according to claim 12, wherein the control portion is further used for executing the following steps:
displaying the blood pressure values and the hypertension degree in a form of a two-dimensional coordinate graph by means of the human-machine interaction device;
or, displaying the arteriosclerosis degree and the hypertension degree in a form of a two-dimensional coordinate graph by means of the human-machine interaction device;
or, displaying at most two of the arteriosclerosis degree and the hypertension degree, and the arterial stiffness index, the blood pressure values and the pulse number in a form of a multidimensional tendency graph by means of the human-machine interaction device;
or, displaying at most four of the blood pressure values, the pulse number, the arteriosclerosis degree and the hypertension degree, and the arterial stiffness index in a form of a dial gauge by means of the human-machine interaction device.

14. The arterial stiffness display apparatus according to claim 10, wherein the control portion is further used for executing the following steps:
acquiring current time;
displaying the current time by means of the human-machine interaction device;
analyzing the vascular age of the user according to the arterial stiffness index;
displaying the vascular age by means of the human-machine interaction device;
generating a curve or histogram of at least one of the arterial stiffness index, the blood pressure value, the pulse number and the vascular age that changes over time;
displaying the curve or histogram in a form of a two-dimensional coordinate graph by means of the human-machine interaction device.

15. The arterial stiffness display apparatus according to claim 10, wherein the control portion is further used for executing the following steps:
acquiring a display mode switching instruction input by the user by means of the human-machine interaction device;
switching a display mode of the human-machine interaction device according to the display mode switching instruction.

16. The arterial stiffness display apparatus according to claim 10, wherein the control portion is further used for executing the following steps:
analyzing the risk, at which the user suffers from a cardiovascular disease, according to the arterial stiffness index, the blood pressure values and the pulse number, and generating an analysis result;
displaying the analysis result by means of the human-machine interaction device.

17. The arterial stiffness display apparatus according to claim 16, further comprising a communication device, wherein the control portion is further used for executing the following steps:
sending the arterial stiffness index, the blood pressure values, the pulse number and the analysis result to a cloud server by means of the communication device, the cloud server being used for sending the arterial stiffness index, the blood pressure values, the pulse number and the analysis result to a client of a doctor, and the doctor diagnosing a disease of the user, and generating a disease diagnosis result and sending the same to the cloud server by means of the client;
receiving the disease diagnosis result sent by the cloud server by means of the communication device;
displaying the disease diagnosis result by means of the human-machine interaction device.

18. A computer-readable storage medium, wherein the computer-readable storage medium stores a computer-readable instruction; the computer-readable instruction realizes, when executed by a processor, the following steps:
acquiring a pressure value of the pressure sensor in a pressure rise or drop process of the arm belt;
generating a pressure characteristic curve of the pressure value changing over time;
acquiring an arterial stiffness index according to a characteristic of a curve segment, corresponding to a preset pressure value, in the pressure characteristic curve;
acquiring blood pressure values and a pulse number according to the pressure value, the blood pressure values comprising a highest blood pressure value and a lowest blood pressure value; and
displaying at most two of the blood pressure values and the pulse number, and the arterial stiffness index by means of the human-machine interaction device.

19. The computer-readable storage medium according to claim 18, wherein the computer-readable instruction further realizes, when executed by the processor, the following steps:
determining whether the arterial stiffness index is greater than a preset arterial stiffness index threshold;
when the arterial stiffness index is greater than the preset arterial stiffness index threshold, sending a prompt that the arterial stiffness index exceeds the standard by means of the human-machine interaction device;
or, determining whether the highest blood pressure value is greater than a preset blood pressure upper limit threshold, and determining whether the lowest blood pressure value is less than a preset blood pressure lower limit value;
when the highest blood pressure value is greater than the preset blood pressure upper limit threshold or the lowest blood pressure value is less than the preset blood pressure lower limit value, sending a prompt that the blood pressure value exceeds the standard by means of the human-machine interaction device;
or, determining whether the pulse number is in a preset pulse number range;
when the pulse number is not in the preset pulse number range, sending a prompt that the pulse number exceeds the standard by means of the human-machine interaction device.

20. The computer-readable storage medium according to claim 18, wherein the computer-readable instruction further realizes, when executed by the processor, the following steps:
determining an arteriosclerosis degree of a user according to the arterial stiffness index;
displaying the arteriosclerosis degree by means of the human-machine interaction device;
determining a hypertension degree of the user according to the blood pressure values;
displaying the hypertension degree by means of the human-machine interaction device.
